(19) Europäisches Patentamt
European Patent Office
Office européen des brevets

(11) EP 2 714 201 B1

(12) EUROPEAN PATENT SPECIFICATION

(45) Date of publication and mention of the grant of the patent:
**19.10.2016 Bulletin 2016/42**

(21) Application number: **12726050.3**

(22) Date of filing: **29.05.2012**

(51) Int Cl.:
***A61K 8/04*** *(2006.01)* ***A61Q 11/00*** *(2006.01)*
***A61K 8/02*** *(2006.01)*

(86) International application number:
**PCT/EP2012/059961**

(87) International publication number:
**WO 2012/163885 (06.12.2012 Gazette 2012/49)**

(54) **COEXTRUDABLE MULTIPHASE DENTIFRICE COMPOSITIONS**

COEXTRUSIONSFÄHIGE MULTIPHASEN-ZAHNCREMEZUSAMMENSETZUNGEN

COMPOSITIONS DE DENTIFRICE MULTIPHASES COEXTRUDABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2011 EP 11168472**

(43) Date of publication of application:
**09.04.2014 Bulletin 2014/15**

(73) Proprietors:
- **Unilever PLC**
  **London, Greater London EC4P 0DY (GB)**
  Designated Contracting States:
  **CY GB IE MT**
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
- **GREEN, Alison, Katharine**
  **Wirral**
  **Merseyside CH63 3JW (GB)**
- **GROVES, Brian, Joseph**
  **Wirral**
  **Merseyside CH63 3JW (GB)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
**EP-A2- 0 138 460**
**WO-A1-2006/081912**
**GB-A- 1 304 090**
**US-A- 4 518 578**

EP 2 714 201 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to coextrudable multiphase dentifrice compositions.

### Background of the Invention

Aesthetics are known to play an important role in consumer choice and use of dentifrice. A unique visual appearance for a dentifrice provides an aesthetic effect that the user finds pleasing and promotes the use of the dentifrice.

In some cases, visual effects such as stripes or particles have been used to distinguish and market new dentifrice products. But there remains a continuous need for new and attractive visual variations for dentifrices.

An example of a visually attractive multiphase dentifrice is provided in WO2006/081912. This describes a multiphase toothpaste composition in which a first phase is disposed co-axially within a second phase. According to this publication, improved visual impact and better, more consistent dispensing from a tube is achieved by raising and carefully configuring the viscosities of each phase. Generally this means that the viscosity of each of the phases is closely matched so that each of the first and second phases has a viscosity within the same narrow range (quoted as from 210 000 to 225 000 mPa.s).

Similarly, WO2006/081933 describes a multiphase toothpaste composition in which a first phase is disposed co-axially within a second phase. In this case, improved visual impact and greater structural stability after dispensing from a tube onto a toothbrush is said to be achieved by carefully configuring the specific densities of each phase. Generally this means that the specific density of each of the phases is closely matched so that each of the first and second phases has a specific density within the same narrow range (quoted as from 1.3 to 1.35 g/ml). WO2007/0999060 is another disclosure of a multiphase toothpaste composition in which a first formulation is located coaxially within a second formulation on dispensing from the container. In this case, it is preferred that the first and second formulations have "substantially similar" viscosities at 40°C (RV-TD at 5 rpm), meaning that the viscosities are kept within 10,000 cps of each other.

us4318578 discloses a visually clear pigmented dentifrice that may be striped and/or may comprise bubbles entrained air.

The present inventors have found that it is possible to selectively introduce entrained air or other gas into one phase of a multiphase dentifrice composition in which a first phase is disposed co-axially within a second phase. Surprisingly, although the entrained gas alters the relative viscosity and density of the phases, it does not appear to adversely affect either the structural stability or the extrudability of the dentifrice composition.

Entrainment of air or other gas in one phase of a dentifrice can enhance certain properties such as visual appeal (e.g. by imparting a pearlescent or metallised appearance to that phase).

The presence of entrained gas in one phase, especially the core, may also enhance the sensory benefits of the dentifrice as perceived by the consumer, such as freshness and flavour delivery.

### Summary of the Invention

The present invention provides a packaged multiphase dentifrice composition in which an inner first phase is in physical contact with and surrounded by a visually distinct outer second phase, and in which the phases are coextrudable from a single dispensing orifice such that the extrudate has a core of inner first phase
and a sheath of outer second phase surrounding the core of inner first phase, characterised in that entrained air or other gas is selectively incorporated into one of the phases, the gas being incorporated as discrete bubbles.

### Detailed Description of the Invention

The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

The dentifrice composition of the invention is a multiphase composition with an inner first phase and an outer second phase arranged in a particular configuration as described above.

Typically, the inner first phase is disposed co-axially within the outer second phase. This generally means that the extruded dentifrice composition (as dispensed from the single dispensing orifice) is in the form of an elongated strip

or ribbon having a generally central longitudinal axis which falls within the inner first phase. Similarly, the dentifrice as stored within the dentifrice packaging has a generally central longitudinal axis which falls within the inner first phase. Such alignment is understood to be judged by the eye and not mathematically.

Preferably, each of the first and the second phases of the dentifrice composition of the invention contains a liquid continuous phase, in an amount of from 40 to 99% by weight based on the total weight of the individual phase (i.e. the first phase or the second phase, taken individually). Such a liquid continuous phase will typically comprise a mixture of water and polyhydric alcohol in various relative amounts, with the amount of water generally ranging from 5 to 45% by weight (based on the total weight of the individual phase) and the amount of polyhydric alcohol generally ranging from 30 to 70% by weight (based on the total weight of the individual phase). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

Most preferably, each of the first and the second phases comprises from 5 to 45% by weight water (based on the total weight of the individual phase) and from 40 to 50% by weight sorbitol (based on the total weight of the individual phase).

Generally, each of the first and the second phases will contain further ingredients to enhance performance and/or consumer acceptability, such as abrasive cleaning agent, binder or thickening agent, and surfactant.

Preferably, each of the first and the second phases also comprises an abrasive cleaning agent in an amount of from 3 to 75% by weight based on the total weight of the individual phase. Suitable abrasive cleaning agents include abrasive silicas such as silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Abrasive silicas are the preferred abrasive cleaning agents in the context of the present invention, and more preferably abrasive silicas with a low refractive index. Examples of such materials include abrasive silicas with an apparent refractive index (R.I.) in the range of 1.41 to 1.47, and preferably 1.435 to 1.445.

Most preferably, each of the first and the second phases comprises an abrasive silica as described above, in an amount ranging from 5 to 15%, such as from 8 to 10%, by weight based on the total weight of the individual phase.

Furthermore, each of the first and the second phases preferably also comprises a binder or thickening agent in an amount of from 0.5 to 10% by weight based on the total weight of the individual phase. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, each of the first and the second phases preferably also comprises a surfactant in an amount of from 0.2 to 5% by weight based on the total weight of the individual phase. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Preferably at least one of the phases is visually clear. More preferably at least the outer second phase is visually clear. Most preferably both the inner first phase and the outer second phase are visually clear. By "visually clear" in the context of this invention it is generally meant that the phase in question is transparent or translucent such that a person with 20:20 eyesight can read a size 12 Times Roman font through a 1 ml cuvette filled with it.

In the dentifrice composition of the invention, entrained air or other gas is selectively incorporated into the internal phase the gas being incorporated as discrete bubbles. By "selectively incorporated" in the context of this invention it is meant that the entrained air or other gas is deliberately incorporated into only one of the phases of the dentifrice composition, namely the internal or inner phase.

The gas may suitably be air or Food Grade gases such as Food Grade Nitrogen or Food Grade 70:30 Nitrogen/Oxygen. The gas is incorporated as discrete bubbles and the bubble size is preferably at least 10 microns and most preferably ranges from 30 to 3500 microns.

The phase which contains the entrained air or other gas (hereinafter referred to as "the aerated phase") is the inner first phase, and the phase which does not contain the entrained air or other gas (hereinafter referred to as "the non-aerated phase") is the outer second phase.

The percentage of air or other gas incorporated in the aerated phase may be defined by measuring the density

of the aerated phase relative to the density of the phase prior to aeration and expressing this as a percentage, as follows:

[density of phase prior to aeration – density of aerated phase] / [density of phase prior to aeration] X 100%

Densities may be measured using a standard density cup.

Generally the percentage of air or other gas in the aerated phase is at least 1 %, more preferably at least 2% and most preferably at least 5%, according to the expression as defined above. Generally the percentage of air or other gas in the aerated phase does not exceed 50%, and more preferably does not exceed 20%, and most preferably does not exceed 10%, according to the expression as defined above.

Preferably the non-aerated phase has a viscosity (as measured on a Brookfield RV TD viscometer at 40°C and 5rpm) ranging from 150 000 cps to 250 000 cps. The aerated phase will generally have a viscosity which is at least 10,000 cps lower than that of the non-aerated phase (when measured by the same protocol) e.g. from 10,000 to 35,000 cps lower.

Generally the proportion of inner first phase relative to the total dentifrice composition (in weight percentage terms) ranges from 2 to 20%, more preferably from 5 to 15% and most preferably from 8 to 12% by total weight inner first phase based on the total weight of the dentifrice composition. For example, preferred dentifrice compositions according to the invention have an amount of inner first phase ranging from 10 to 20% and an amount of outer second phase ranging from 80 to 90%, by total weight of inner first phase or outer second phase respectively, based on the total weight of the dentifrice composition.

In a typical manufacturing process for the dentifrice composition according to the invention the inner first phase and outer second phase are manufactured separately and then coextruded into their package by equipment such as that described in WO99/01342, i.e. a coaxial nozzle assembly attached to standard equipment.

The phases of the dentifrice composition may suitably be manufactured by conventional mixing processes known to those skilled in the art.

Incorporation of the entrained air or other gas into one of the phases may suitably be achieved by metering the desired gas into a high shear mixer in admixture with the remaining ingredients of the selected phase (which is preferably the inner first phase). For example, incorporation of the entrained air or other gas can be achieved at the point of manufacture of the selected phase using a conventional batch-processing vacuum mixer (e.g. a Fryma® VME) which has been adapted with the addition of a gas injection point. The desired quantity of gas can be metered via the gas injection point into the base of the mixer and the desired bubble size controlled via shear generated by the mixer and the residence time. Alternatively, aeration may be achieved in a conventional batch-processing vacuum mixer without any gas injection, by closing and holding the vacuum, drawing atmospheric air in to the vessel through the bottom inlet valve and metering the quantity of air added (by using the drop on the vacuum gauge). This method has the advantage that no modifications of equipment, compressors or compressed gas canisters are necessary. Also, if excess air is accidentally incorporated, this is easily rectifiable by de-gassing and starting again. Incorporation of the entrained air or other gas can also be achieved post-manufacture of the selected phase (which is preferably the inner first phase) via a continuous or semi-continuous route, in which the pre-manufactured phase is pumped through pipework containing a gas injection point for metered gas inclusion and then passed through static mixers to give an initial dispersion, followed by high shear mixing (e.g. with a Silverson® mixer) to achieve the desired bubble size. Suitable equipment to perform such a process is known to the skilled worker and commercially available (for example from Kinematica®)

The package is typically a tubular container comprising a tubular body which is crimped at one end and comprises a single dispensing orifice at the other end, through which the phases of the dentifrice composition are coextruded by the consumer.

Dentifrice compositions of the present invention may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

A preferred class of optional ingredient for use in the invention are colouring agents. The inner, first phase and outer, second phase may for example contain different types and/or amounts of colouring agents so as to provide attractive visual effects.

A preferred class of colouring agents for use in the present invention includes pigments. The term "pigment" is generally understood to mean any organic or inorganic material which exhibits absorption at a wavelength ranging from 350 nm to 700 nm, preferably an absorption with a maximum, and whose solubility in water is less than 0.01 g, preferably less than 0.001 g, more preferably less than 0.0001 g, of material dissolved per 100 g of water at 20°C.

Examples of pigments which may used include organic pigments. Suitable organic pigments include monoazo pigments, benzimidazolone pigments, disazo condensation pigments, pyrazolone pigments, phthalocyanine pigments,

quinacridone pigments, perylene pigments, perinone pigments, anthraquinone pigments, indanthrone pigments, dioxazine pigments and thioindigo pigments and mixtures thereof.

Examples of preferred pigments in the context of the present invention include monoazo pigments, phthalocyanine pigments and thioindigo pigments and mixtures thereof.

Suitable monoazo pigments include azo derivatives of beta-naphthol and azo derivatives of 2-hydroxy-3-naphthoic acid (also called Naphthol AS pigments). Examples of such materials include those having the C.I. generic name Pigment Red 4 (C.I. constitution number 12085) or the C.I. generic name Pigment Red 5 (C.I. constitution number 12490) respectively. A commercially available example is Cosmenyl Red-R, ex Clariant.

Suitable thioindigo pigments include halogenated derivatives of thioindigo. An example of such a material has the C.I. generic name Pigment Red 181 (C.I. constitution number 73360), and is commercially available as Rouge Covasorb W 3790, ex Sensient Cosmetic Technologies/LCW.

The most preferred pigments in the context of the present invention are phthalocyanine pigments, and in particular phthalocyanine blue or phthalocyanine green pigments. Phthalocyanines are organometallic compounds containing four symmetrically arranged isoindole rings connected in a 16-membered ring linking with alternate carbon and nitrogen atoms. Most phthalocyanines contain a central, co-ordinated metal ion such as copper. Copper phthalocyanines have the basic structure:

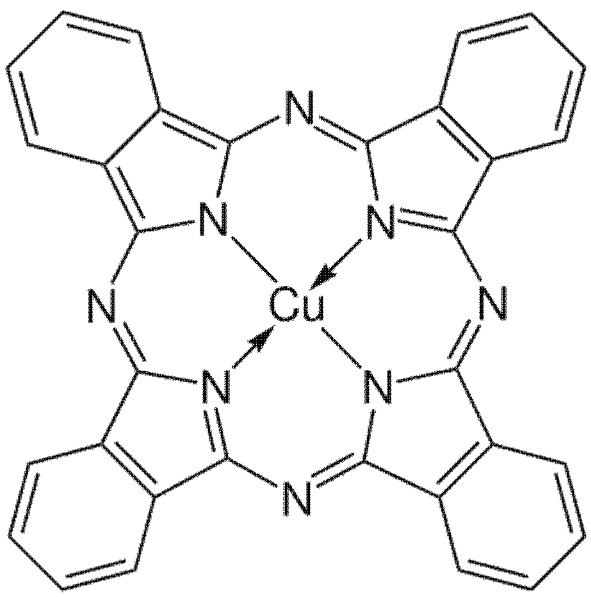

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule.

Most phthalocyanine green pigments are copper polyhalophthalocyanines, such as copper polychlorophthalocyanines or copper polybromochlorophthalocyanines. Polyhalogenated green copper phthalocyanine pigments are not polymorphous.

The following pigments are illustrative phthalocyanine blue and phthalocyanine green pigments:

| C.I. Generic Name | C.I. Constitution Number | Crystal modification; Type | Number of halogen atoms |
|---|---|---|---|
| Pigment Blue 15 | 74 160 | alpha | - |
| Pigment Blue 15:1 | 74 160 | alpha; phase stabilised | 0.5-1 Cl |
| Pigment Blue 15:2 | 74 160 | alpha; phase & flocculation stabilised | 0.5-1 Cl |
| Pigment Blue 15:3 | 74 160 | beta | - |
| Pigment Blue 15:4 | 74 160 | beta; flocculation stabilised | - |

(continued)

| C.I. Generic Name | C.I. Constitution Number | Crystal modification; Type | Number of halogen atoms |
|---|---|---|---|
| Pigment Blue 15:6 | 74 160 | epsilon | - |
| Pigment Blue 16 | 74 100 | gamma; metal-free | - |
| Pigment Green 7 | 74 260 | | 14-15 Cl |
| Pigment Green 36 | 74 265 | | 4-9 Br; 8-2 Cl |

The most preferred phthalocyanine blue pigments for use in the invention are those alpha copper phthalocyanines which are designated as C.I. Pigment Blue 15, C.I. Pigment Blue 15:1 or C.I. Pigment Blue 15:2 respectively, as described in the above Table. A commercially available example is Covarine Blue W 6795, ex Sensient Cosmetic Technologies/LCW.

The most preferred phthalocyanine green pigments for use in the invention are those copper polychlorophthalocyanines which are designated as C.I. Pigment Green 7, as described in the above Table. A commercially available example is Cosmenyl Green GG, ex Clariant.

Mixtures of any of the above described materials may also be used.

In a preferred embodiment, a colouring agent (such as the phthalocyanine pigments described above) is selectively incorporated into one of the phases, such as the inner first phase. In a particularly preferred embodiment, the outer second phase is substantially colourless and visually clear, and the inner first phase comprises from 0.001 to 0.2%, preferably from 0.001 to 0.01%, of one or more colouring agents (by total weight colouring agent based on the total weight of the inner first phase).

The invention is further illustrated with reference to the following, non-limiting examples. All concentrations are expressed by weight percent of the total formulation, and as level of active matter.

**EXAMPLE**

Formulations were prepared having ingredients as shown in the following Table:

| Ingredient | Formula A (%w/w) | Formula B (%w/w) |
|---|---|---|
| Silica abrasive | 8 | 8 |
| Silica thickening | 8 | 8 |
| Sodium monofluorophosphate | 0.8 | 0.8 |
| PEG 1500 | 4 | 4 |
| Sorbitol syrup (70% a.i.) | 63 | 63 |
| Flavour | 1.1 | 1.1 |
| Saccharin | 0.2 | 0.2 |
| Trisodium phosphate | 0.1 | 0.1 |
| Cellulose gum | 0.9 | 0.9 |
| Sodium lauryl sulphate | 2 | 2 |
| Cosmenyl Green GG (Colour Index 74260) | - | 0.005 |
| Water | to 100% | to 100% |

Formula B was aerated with 6% air (the percentage of air being calculated as described above). After aeration, the viscosity of Formula B was 196800 cps (averaged over 4 measurements on a Brookfield RV TD viscometer at 40°C and 5rpm). Formula A was not aerated and its viscosity was 208700 cps (when measured according to the same protocol).

Non-aerated Formula A and aerated Formula B were then coextruded into a conventional dentifrice packaging tube having a single dispensing orifice, using a coaxial nozzle assembly. The resulting packaged multiphase dentifrice composition according to the invention has a core (of aerated Formula B) which is surrounded by a sheath (of non-aerated Formula A). The composition as stored within the tube has a generally central longitudinal axis which falls within the core. The composition has 10% core (by weight based on the total weight of the composition) and 90% sheath (by weight based on the total weight of the composition).

A strip of the above composition was extruded onto the bristles of a toothbrush. Figure 1 is a photograph of the extruded strip on the toothbrush. Discrete bubbles are clearly visible in the core. Furthermore, this configuration is stably maintained, with no apparent loss of aeration, even after over 8 weeks storage at room temperature.

## Claims

1. A packaged multiphase dentifrice composition in which an inner first phase is in physical contact with and surrounded by a visually distinct outer second phase, and in which the phases are coextrudable from a single dispensing orifice such that the extrudate has a core of inner first phase and a sheath of outer second phase surrounding the core of inner first phase, **characterised in that** entrained air or other gas is selectively incorporated into the internal phase ("the aerated phase") the gas being incorporated as discrete bubbles and the phase which does not contain the entrained air or other gas ("the non-aerated phase") is the outer second phase

2. A composition according to claim 1, in which the inner first phase is disposed co-axially within the outer second phase.

3. A composition according to claim 1 or claim 2, in which each of the first and the second phases comprises from 5 to 45% by weight water (based on the total weight of the individual phase) and from 40 to 50% by weight sorbitol (based on the total weight of the individual phase).

4. A composition according to any one of claims 1 to 3, in which both the inner first phase and the outer second phase are visually clear.

5. A composition according to any one of claims 1 to 4, in which the percentage of air or other gas in the aerated phase ranges from 5% to 10%, when calculated according to the expression:

$$[\text{density of phase prior to aeration} - \text{density of aerated phase}] / [\text{density of phase prior to aeration}] \times 100\%.$$

6. A composition according to any one of claims 1 to 5, in which the outer second phase is substantially colourless and visually clear, and the inner first phase comprises from 0.001 to 0.2% of one or more colouring agents (by total weight colouring agent based on the total weight of the inner first phase).

7. A composition according to claim 6, in which the colouring agent is a phthalocyanine blue or a phthalocyanine green pigment.

8. A composition according to any one of claims 1 to 7, in which the package is a tubular container comprising a tubular body which is crimped at one end and comprises the single dispensing orifice at the other end, through which the phases of the dentifrice composition may be coextruded.

## Patentansprüche

1. Verpackte mehrphasige Zahncremezusammensetzung, bei der eine innere erste Phase mit einer visuell unterschiedlichen äußeren zweiten Phase in physischem Kontakt und von dieser umgeben ist, und bei der die Phasen aus einer einzige Ausgabeöffnung gemeinsam so herausgedrückt werden können, dass der Strang einen Kern aus einer inneren ersten Phase und eine Ummantelung aus einer äußeren zweiten Phase, die den Kern der inneren ersten Phase umgibt, aufweist, **dadurch gekennzeichnet, dass** eingeschlossene Luft oder ein anderes Gas wahlweise in der inneren Phase ("der mit Luft durchsetzten Phase") enthalten ist, wobei das Gas als einzelne Blasen enthalten ist und wobei die Phase, die die eingeschlossene Luft oder ein anderes Gas nicht enthält ("die nicht mit Luft durchsetzte Phase"), die äußere zweite Phase bildet.

2. Zusammensetzung nach Anspruch 1, wobei die innere erste Phase koaxial mit der äußeren zweiten Phase angeordnet ist.

3. Zusammensetzung nach Anspruch 1 oder Anspruch 2, wobei die erste und die zweite Phase Wasser im Bereich von 5 bis 45 Gew.-% (basierend auf dem Gesamtgewicht der einzelnen Phase) und Sorbitol im Bereich von 40 bis 50 Gew.-% (basierend auf dem Gesamtgewicht der einzelnen Phase) umfassen.

4. Zusammensetzung nach einem der Ansprüche 1 bis 3, wobei sowohl die innere erste Phase als auch die äußere zweite Phase durchsichtig sind.

5. Zusammensetzung nach einem der Ansprüche 1 bis 4, wobei der Prozentsatz von Luft oder einem anderen Gas in der mit Luft durchsetzten Phase im Bereich von 5 % bis 10 % liegt, wenn er nach dem folgenden Ausdruck berechnet wird:

[Dichte der Phase vor einem Durchsetzen mit Luft – Dichte der mit Luft durchsetzten Phase] / [Dichte der Phase vor einem Durchsetzen mit Luft] × 100 %.

6. Zusammensetzung nach einem der Ansprüche 1 bis 5, wobei die äußere zweite Phase im Wesentlichen farblos und durchsichtig ist und wobei die innere erste Phase ein oder mehrere Färbemittel im Bereich von 0,001 bis 0,2 % (Gesamtgewicht von Färbemittel bezogen auf das Gesamtgewicht der inneren ersten Phase) umfasst.

7. Zusammensetzung nach Anspruch 6, wobei das Färbemittel ein Phthalocyaninblau-Pigment oder ein Phthalocyaningrün-Pigment ist.

8. Zusammensetzung nach einem der Ansprüche 1 bis 7, wobei die Verpackung ein schlauchförmiger Behälter ist, der einen schlauchförmigen Körper umfasst, der an einem Ende gefalzt ist und die einzige Ausgabeöffnung an dem anderen Ende umfasst, durch die die Phasen der Zahncremezusammensetzung zusammen herausgedrückt werden können.

## Revendications

1. Composition dentifrice conditionnée en plusieurs phases, dans laquelle une première phase intérieure est en contact physique avec une deuxième phase extérieure visuellement distincte et entourée par cette dernière, et dans laquelle les phases peuvent être coextrudées à partir d'un orifice distributeur unique, de telle sorte que l'extrudat possède un coeur en la première phase intérieure et une gaine en la deuxième phase extérieure, entourant le coeur en la première phase intérieure, **caractérisée en ce que** de l'air ou un autre gaz entraîné est sélectivement incorporé dans la phase interne ("la phase aérée"), le gaz étant incorporé sous forme de bulles discrètes, et la phase qui ne contient pas l'air ou l'autre gaz entraîné ("la phase non aérée") étant la deuxième phase extérieure.

2. Composition selon la revendication 1, dans laquelle la première phase intérieure est disposée coaxialement à l'intérieur de la deuxième phase extérieure.

3. Composition selon la revendication 1 ou la revendication 2, dans laquelle chacune de la première et de la deuxième phases comprend de 5 à 45 % en poids d'eau (par rapport au poids total de la phase individuelle) et de 40 à 50 % en poids de sorbitol (par rapport au poids total de la phase individuelle).

4. Composition selon l'une quelconque des revendications 1 à 3, dans laquelle tant la première phase intérieure que la deuxième phase extérieure sont visuellement limpides.

5. Composition selon l'une quelconque des revendications 1 à 4, dans laquelle le pourcentage d'air ou d'un autre gaz dans la phase aérée est compris dans la plage de 5 % à 10 %, le calcul étant effectué selon l'expression [masse volumique de la phase avant aération - masse volumique de la phase aérée] / [masse volumique de la phase avant aération] X 100 %.

**6.** Composition selon l'une quelconque des revendications 1 à 5, dans laquelle la deuxième phase extérieure est sensiblement incolore et visuellement limpide, et la première phase intérieure comprend de 0,001 à 0,2 % d'un ou plusieurs agents colorants (en poids total de l'agent colorant, par rapport au poids total de la première phase intérieure).

**7.** Composition selon la revendication 6, dans laquelle l'agent colorant est un bleu de phtalocyanine ou un pigment vert de phtalocyanine.

**8.** Composition selon l'une quelconque des revendications 1 à 7, dans laquelle le conditionnement est un récipient tubulaire comprenant un corps tubulaire qui est serti à une extrémité et comprend l'orifice distributeur unique à l'autre extrémité, par lequel les phases de la composition dentifrice peuvent être coextrudées.

Fig. 1

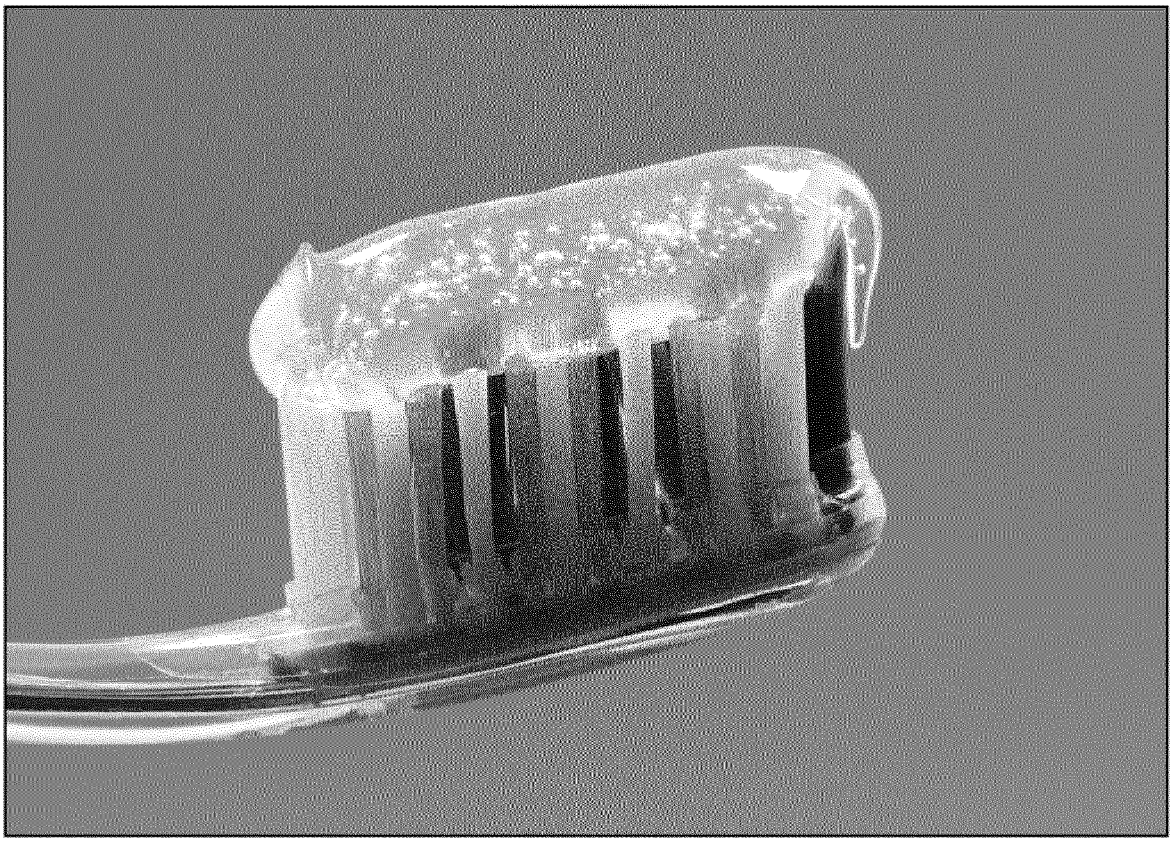

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006081912 A
- WO 2006081933 A
- WO 20070999060 A
- WO 9901342 A